# EUROPEAN PATENT APPLICATION

(11) **EP 1 829 534 A1**
(43) Date of publication of application: **05.09.2007**
(21) Application number: 06384003.7
(22) Date of filing: 02.03.2006
(51) Int. Cl.: A61K 31/00, A61P 3/00, A61P 3/06, A61P 3/04

(54) **Use of compounds binding to the sigma receptor for the treatment of metabolic syndrome**

(71) Applicant: LABORATORIOS DEL DR. ESTEVE, S.A., 08041 Barcelona (ES)
(72) Inventor: Buschmann, Helmut H., 08960 Sant Just Desvern (ES); Vela Hernandez, Jose Miguel, 08041 Barcelona (ES)
(74) Representative: Peters, Hajo

(57) **Abstract**

The present invention refers to the use of compounds binding to the sigma receptor for the treatment of metabolic syndrome.

## Description

### Field of the invention

The present invention refers to the use of compounds binding to the sigma receptor for the treatment of metabolic syndrome, especially hyperlipidemias, in particular hypertriglyceridemias and the prevention or the prophylaxis of the symptoms of metabolic syndrome, especially hyperlipidemias, in particular hypertriglyceridemias.

### Background of the invention

The treatment of metabolic syndrome is of great importance in medicine. The metabolic syndrome is a widespread disease, particularly in the United States and Europe. Based on survey data from 1988 to 1994 and 2000 census data, the American Center for Disease Control and Prevention estimates that 47 million people in the US have metabolic syndrom. There is currently a world-wide need for treatment of this syndrome as it is identified as heightening the risk of cardiovascular mortality.

Consequently, it was an object of the present invention to provide medicaments, which are suitable for the treatment of metabolic syndrome.

Therefore, it was the underlying problem solved by this invention to find new ways of treating metabolic syndrome.

So, the main object of this invention is the use of a compound binding to the sigma receptor in the production of a medicament for the treatment of metabolic syndrome.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.

The metabolic syndrome is characterized by an interaction of several physiological parameters such as triglycerides, lipids, blood pressure, glucose levels and insuline levels. Thus it includes especially hyperlipidemias and hypertriglyceridemia.

Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used compounds binding to the sigma receptor.

Hypertriglyceridemia can be categorized by the Fredrickson classification of lipid disorders (Fredrickson, 1971; Beaumont et al., 1970). All hyperlipidemias (types I, IIb, III, IV and V) except type IIa are characterized by elevated triglyceride levels. Thus, the present invention claims the use of sigma-1 receptor antagonists for treating the following types of hypertriglyceridemias:
- **Type I:** It is characterized by severe elevations in chylomicrons and elevated triglycerides. Because chylomicrons also contain a small amount of cholesterol, serum cholesterol levels also are quite high.
- **Type IIb:** It is the classic mixed hyperlipidemia (high cholesterol and triglycerides) caused by elevations in both low-density lipoprotein (LDL) and very low-density lipoprotein (VLDL).
- **Type III:** It is also known as dysbetalipoproteinemia, remnant removal disease, or broad-beta disease. Typically, these patients have elevated total cholesterol and triglyceride levels and are easily confused with patients with type IIb hyperlipidemia. Patients with type III hyperlipidemia have elevations in intermediate-density lipoprotein (IDL), a VLDL remnant.
- **Type IV:** It is characterized by abnormal elevations of VLDL and triglycerides. Serum cholesterol levels are normal.
- **Type V:** It is the combination of types I and IV (elevations of both chylomicrons and VLDL). Serum cholesterol levels always are elevated, but the LDL cholesterol levels are normal. Given the rarity of type I disease, when elevated triglycerides are noted, the most likely cause is type V hyperlipidemia.

This/these compound/s may be in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable mixing ratio.

While working on compounds binding to the sigma receptor and with models like knock-out mice it was surprisingly found out that metabolic syndrome is connected to the sigma receptor so that compounds binding to the sigma receptor were acting on metabolic syndrome with a high potency.

The term "treatment" as used in the present application encompasses prevention, amelioration and/or complete recovery from the disease. Said term also includes the prevention, amelioration and/or complete recovery of one or more symptoms associated with the disease.

"The sigma receptor/s" as used in this application is/are well known and defined using the following citation: This binding site represents a typical protein different from opioid, NMDA, dopaminergic, and other known neurotransmitter or hormone receptor families (G. Ronsisvalle et al. Pure Appl. Chem. 73, 1499-1509 (2001)). Pharmacological data based on ligand binding studies, anatomical distribution and biochemical features distinguish at least two subtypes of σ receptors (R. Quiron et al., Trends Pharmacol. Sci. 13, 85-86 (1992); M.L.Leitner, Eur. J. Pharmacol. 259, 65-69 (1994); S.B. Hellewell and W.D. Bowen; Brain Res. 527, 244-253 (1990)) (G. Ronsisvalle et al. Pure Appl. Chem. 73, 1499-1509 (2001)). The protein sequence of sigma receptors (Sigma 1 (σ1) and Sigma 2 (σ2)) is known (e.g. Prasad, P.D. et al., J. Neurochem. 70 (2), 443-451 (1998)) and they show a very high affinity for e.g. pentazocine.

"Compound/s binding to the sigma receptor" or "sigma ligand" as used in this application is/are defined as having an IC₅₀ value of ≤5000 nM, more preferably ≤1000 nM, more preferably ≤500 nM. More preferably, the IC₅₀ value is ≤50 nM. More preferably, the IC₅₀ value is ≤100 nM. Most preferably, the IC₅₀ value is ≤ 50 nM. Additionally, the wording "Compound/s binding to the sigma receptor", as used in the present application is defined as having at least ≥50% displacement using 10 mM radioligand specific for the sigma receptor (e.g. preferably ¹H-pentazocine) whereby the sigma recepor may be any sigma receptor subtype. Preferably, said compounds bind to the sigma-1 receptor subtype.

Compounds binding to the sigma receptor generally also known as sigma ligands are well known in the art with many of them falling under the definition for "Compound/s binding to the sigma receptor" set up above. Still even though there are many uses known for sigma ligands such as antipsychotic drugs, anxiolytics, antidepressants, the treatment of stroke, antiepileptic drugs and many other indications there is nowhere any mentioning of these compounds being useful against metabolic syndrome.

Compounds binding to the sigma receptor known in the art and matching the criteria of sigma ligand (i.e. having an IC₅₀ ≤ 5000 nM) as mentioned above, are listed below. Some of these compounds may bind to the sigma-1 and/or the sigma-2 receptor. Preferably, these compounds are in form of a salt, a base or an acid. Also preferably, the salts/bases/acids indicated in the list are to be understood as being exemplary and therefore may respresent any salt, base or acid of the compound.

| (-)-Cyanopindolol hemifumarate | (-)-SPARTEINE SULFATE PENTAHYDRATE |
|---|---|
| (+)-HIMBACINE | (2-Dibutylamino-Ethyl)-Carbamic Acid 2-(4-Benzofuran-2-Ylmethyl-Piperazin-1-Yl)-Ethyl Ester |
| (4-[1,2,3]Thiadiazol-4-Yl-Benzyl)-Carbamic Acid 1-(3-Methoxy-2-Nitro-Benzyl)-Piperidin-3-Ylmethyl Ester | (S)-Methamphetamine HCl |
| [1-(9-Ethyl-9H-Carbazol-3-Ylmethyl)-Pyrrolidin-3-Yl]-Carbamic Acid 1-(3-Benzyloxy-4-Methoxy-Benzyl)-Piperidin-3-Ylmethyl Ester | [1-(9-Ethyl-9H-Carbazol-3-Ylmethyl)-Pyrrolidin-3-Yl]-Carbamic Acid 2-(Tert-Butoxycarbonyl-Naphthalen-1-Ylmethyl-Amino)-Ethyl Ester |
| [4-(4-Ethy)-3,5-Dimethyl-Pyrazol-1-Yl)- Phenyl]-[4-(3-Phenyl-Allyl)-Piperazin-1- YI]-Methanone | 1-(1,2-Diphenylethyl)Piperidine Maleate, (+/-) |
| 1-(1-Naphthyl)Piperazine HCl | 1-(3-Chlorophenyl)Piperazine HCl |
| 1-(4-Bromo-Benzenesulfonyl)-4-(2-Tert- | 2-(2-{[1-(3-Chloro-Benzyl)-Pyrrolidin- |
| Butylsulfanyl-Benzyl)-Piperazine | 3-YI]-Methyl-Carbamoyl}-2-Methyl-Propyl)-4,6-Dimethyl-Benzoic Acid |
| 2-Chloro-11-(4-Methylpiperazino)Dibenz[B, F]Oxepin Maleate | 3,3'-Diethylthiacarbocyanine Iodide |
| 3-Mercapto-2-Methylpropanoic Acid 1,2- Diphenylethylamine Salt | 3-Quinuclidinyl Benzilate |
| 3-Tropanyl-3,5-Dichlorobenzoate | 3-Tropanyl-Indole-3-Carboxylate HCl |
| 4-(1 H-Indol-4-YI)-Piperazine-1-Carboxylic Acid 2-(5-Bromo-2-Ethoxy-Phenylamino)-Cyclohexylmethyl Ester | 4-(2-Tert-Butylsulfanyl-Benzyl)-Piperazine-1-Carboxylic Acid 2-Thiophen-2-YI-Ethyl Ester |
| 4-(3,5-Dimethoxy-Phenyl)-Piperazine-1-Carboxylic Acid 1-(2-Fluoro-Benzyl)-Piperidin-2-Ylmethyl Ester | 4-(3-Nitro-5-Sulfamoyl-Thiophen-2-YI)-Piperazine-1-Carboxylic Acid 1-(2-Fluoro-5-Methoxy-Benzyl)-Piperidin-3-Ylmethyl Ester |
| 4-(4-Fluorobenzoyl)-1-(4-Phenylbutyl)Piperidine Oxalate | 4-(5-Trifluoromethyl-Pyridin-2-Yl)-Piperazine-1-Carboxylic Acid Pent-2-Ynyl Ester |
| 4,4'-Bis[4-(P-Chlorophenyl)-4-Hydroxypiperidino]Butyrophenone | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3-Ylmethyl)-Pyrrolidin-3-Yl]-2-Trifluoromethoxy-Benzenesulfonamide | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | 4-Methoxy-N-[1-(7-Methoxy-Benzo[1,3]Dioxol-5-Ylmethyl)-Pyrrolidin-3-Yl]-Benzenesulfonamide |
| 5-(N-Ethyl-N-Isopropyl)-Amiloride | 7-Hydroxy-DPAT HBr, (±)- |
| 8-Hydroxy-DPAT HBr, (R)-(+)- | 8-Hydroxy-DPAT HBr, S(-)- |
| 9-[4-({[4'-(trifluoromethyl)-1.1 1'-biphenyl-2-yl]carbonyl}amino)piperidin-1-yl]-N-(2,2,2-trifluoroethyl)-9H-fluorene-9-carboxamide | Acepromazine Maleate |
| Acetophenazine Maleate | Acrinol |
| Ajmaline | Alaproclate HCl |
| Aloe-Emodin | Alprenolol D-Tartrate Salt Hydrate |
| Alprenolol HCl | AMI-193 |
| Aminobenztropine | Amiodarone HCl |
| Amodiaquine HCl | Amorolfine HCl |
| Amoxapine | Anileridine HCl |
| Anisotropine Methylbromide | Anpirtoline |
| ARC 239 DiHCl | Astemizole |
| Auramine O HCl | Azaperone |
| Azatadine Maleate | Azelastine HCl |
| Bamethan sulfate | BD 1008 DiHBr |
| BD-1047 | BD-1063 |
| Benextramine TetraHCl | Benfluorex HCl |
| Benidipine HCl | Benoxathian HCl |
| Benoxinate HCl | Benperidol |
| Benproperine Phosphate | Benzododecinium bromide |
| Benzphetamine HCl | Benztropine Mesylate |
| Benzydamine HCl | Bephenium Hydroxynaphthoate |
| Bepridil HCl | Berberine chloride |
| Betaxolol HCl | Bifemelane |
| BMY 7378 DiHCl | Bopindolol Malonate |
| BP 554 Maleate | Bromhexine HCl |
| Bromodiphenhydramine HCl | Bromperidol |
| Brompheniramine Maleate | BTCP HCl |
| Buclizine HCl | Buflomedil HCl |
| Bupropion HCl | Buspirone HCl |
| Butacaine Sulfate | Butaclamol HCl, (±)- |
| Butenafine HCl | Butoconazole Nitrate |
| BW 723C86 HCl | Carbetapentane Citrate |
| Carbinoxamine Maleate | Carpipramine DiHCl DiH2O |
| Carvedilol | Cephapirin Benzathine |
| CGS-12066A Maleate | Chloroprocaine HCl |
| Chloroquine Phosphate | Chlorpheniramine Maleate |
| Chlorphenoxamine HCl | Chlorpromazine HCl |
| Chlorprothixene | Cinanserin HCl |
| Cinnarizine | Cirazoline HCl |
| Cis-(+/-)-N-Methyl-N-[2-(3,4-Dichlorophenyl)Ethyl]-2-(1-Pyrrolidinyl)Cyclohexamine DiHBr | Cis(Z)-Flupentixol DiHCl |
| Cisapride Hydrate | Citalopram HBr |
| Clebopride Maleate Salt | Clemastine Fumarate |
| Clemizole HCl | Clenbuterol HCl |
| Clidinium Bromide | Clobenpropit 2HBr |
| Clofazimine | Clofilium Tosylate |
| Clomiphene Citrate | Clomiphene Related Compound A |
| Clomipramine | Cloperastine HCl |
| Clorgyline HCl | Clozapine |
| CONESSINE | Cyclizine |
| Cyclobenzaprine HCl | Cycloheximide |
| Cyproheptadine HCl | Darrow Red HCl |
| Demecarium Bromide | Denatonium Benzoate |
| Deptropine Citrate | Desloratadine |
| Dexbrompheniramine Maleate | Dexchlorpheniramine Maleate |
| Dexfenfluramine HCl | Dibucaine HCl |
| Dicyclomine HCl | Diethylpropion HCl |
| Dimethisoquin HCl | Dimetindene Maleate |
| Diphemanil Methylsulfate | Diphenidol HCl |
| Diphenoxylate HCl | Diphenylpyraline HCl |
| Dipropyldopamine HBr | Dobutamine HCl |
| Donepezil HCl | Doxepin HCl |
| Droperidol | Duloxetine |
| Dyclonine HCl | Ebastine |
| Econazole Nitrate | Epinastine HCl |
| Ethaverine HCl | Ethopropazine HCl |
| Eticlopride HCl, S(-)- | Etofenamate |
| Etonitazenyl Isothiocyanate | Femoxetine HCl |
| Fenfluramine HCl | Fentanyl Citrate |
| Fenticonazole Nitrate | Fipexide HCl |
| Flavoxate HCl | Flunarizine diHCl |
| Fluoxetine Related Compound B | Fluperlapine |
| Fluphenazine Decanoate DiHCl | Fluphenazine Enanthate DiHCl |
| Fluphenazine HCl | Fluphenazine N-Mustard DiHCl |
| Flurazepam Related Compound C | Fluspirilene |
| Fluvoxamine Maleate | GBR 12783 DiHCl |
| GBR 12909 DiHCl | GBR 13069 DiHCl |
| GBR-12935 DiHCl | GR 89696 Fumarate |
| Guanabenz Acetate | Guanadrel Sulfate |
| Guanethidine Sulfate | Halofantrine HCl |
| Haloperidol | HEAT HCl |
| Hexylcaine HCl | Hycanthone |
| Hydroxychloroquine Sulfate | Hydroxyzine HCl |
| Hyoscyamine Sulfate | IBZM, S(-)- |
| ICI-199,441 HCl | Ifenprodil Tartrate |
| Imipramine HCl | Indatraline HCl |
| lofetamine HCl | Irinotecan HCl |
| Isamoltane Hemifumarate | Isopromethazine HCl |
| Isoxsuprine HCl | Ketanserin L-Tartrate |
| Ketoconazole | Ketotifen Fumarate Salt |
| L-693,403 Maleate | L-741,626 |
| L-741,742 HCl | L-745,870 TriHCl |
| Labetalol HCl | Levetimide HCl, R(-) |
| Levobunolol HCl | Lidoflazine |
| Lisuride Hydrogen Maleate, R(+)- | Lobeline HCl |
| lomerizine diHCl | Loperamide HCl |
| Loxapine Succinate | LY-53,857 Maleate |
| Maprotiline HCl | Mazindol |
| MDL 12,330A HCl | Mebhydroline 1,5-naphthalendisulfonate Salt |
| Meclizine HCl | Mefloquine HCl |
| Meprylcaine HCl | Mesoridazine Besylate |
| Metaphit Methanesulfonate | Metergoline |
| Methantheline Bromide | Methdilazine |
| Methiothepin Mesylate | Methixene HCl |
| Methoctramine | Methotrimeprazine Maleate |
| Methylene Violet 3Rax HCl | Metipranolol |
| Mexiletine HCl | Mianserin HCl |
| Miconazole | ML-9 HCl |
| Morantel Hydrogen L-Tartrate | MR 16728 HCl |
| N-(2-Chloroethyl)-N-Ethyl-2-Bromobenzylamine HCl | N'-[2-(Benzo[1,2,5]Thiadiazole-4-Sulfonylamino)-Acetyl]- |
| | Hydrazinecarboxylic Acid 2-(2-{4-[(4-Chloro-Phenyl)-Phenyl-Methyl]-Piperazin-1-Yl}-Ethoxy)-Ethyl Ester |
| Nafronyl Oxalate Salt | Naftifine |
| Naftopidil diHCl | Naltriben Mesylate |
| NAN-190 HBr | NE-100 |
| Nefazodone | Nefopam HCl |
| Nicardipine HCl | Nicergoline |
| Niguldipine HCl, (+/-)- | Nisoxetine HCl |
| Nortriptyline HCl | Nylidrin HCl |
| Octoclothepin Maleate, (±)- | Orphenadrine Citrate |
| Oxamniquine | Oxamniquine Related Compound A |
| Oxamniquine Related Compound B | Oxatomide |
| Oxiconazole Nitrate | Oxybutynin HCl |
| Panaxatriol | PAPP |
| Paroxetine | Paxilline |
| p-Chlorobenzhydrylpiperazine | Penbutolol Sulfate |
| Pentamidine Isethionate | Pentazocine, (±)- |
| Pergolide Methanesulfonate | Perhexiline Maleate Salt |
| Perospirone | Perphenazine |
| Perphenazine Sulfoxide | Phenamil Methanesulfonate |
| Phencyclidine HCl | Phenosafranin HCl |
| Phenoxybenzamine HCl | Phenyltoloxamine Citrate Salt |
| Piboserod | Pimozide |
| Pinacyanol Chloride | Pindobind, (+/-)- |
| Piperacetazine | Piperazine-1,4-Dicarboxylic Acid Benzyl Ester 2-[4-(4-Dimethylamino-Benzyl)-Piperazin-1-Yl]-Ethyl Ester |
| Piperidolate HCl | Pirenperone |
| PPHT HCl, (±)- | Pramoxine HCl |
| Prenylamine Lactate Salt | Pridinol Methanesulfonate Salt |
| Prochlorperazine Maleate | Procyclidine HCl |
| Proflavine Hemisulfate Salt | Progesterone |
| Promazine HCl | Promethazine HCl |
| Propafenone HCl | Proparacaine HCl |
| Propericyazine | Propiomazine |
| Propranolol HCl | Protokylol |
| Protriptyline HCl | Pyrilamine Maleate |
| Pyrimethamine | Pyrrolidine-1,2-Dicarboxylic Acid 1-[1-(4-Allyloxy-Benzyl)-Piperidin-2-Ylmethyl] Ester 2-Benzyl Ester |
| Pyrvinium Pamoate | Quetiapine Fumarate |
| Quinacrine HCl | Quinaldine Red |
| Quipazine Dimaleate | Quipazine, 6-Nitro-, Maleate |
| Raloxifene | Rimantadine HCl |
| Risperidone | Ritanserin |
| Ritodrine HCl | RS 23597-190 HCl |
| RS 67333 HCl | RS 67506 HCl |
| Safranin O HCl | Salmeterol |
| SB203186 | SCH-23390 HCl, R(+)- |
| Sertaconazole Nitrate | Sertindole |
| Sertraline | Sibutramine HCl |
| SKF-525A HCl | SKF-96365 HCl |
| SNC 121 | Spiperone HCl |
| Sufentanil | T-226296 |
| Tamoxifen Citrate | Tamsulosin HCl |
| Tegaserod Maleate | Terbinafine HCl |
| Terconazole | Terfenadine |
| Terfenadine Related Compound A | Tetracaine HCl |
| Tetrindole Mesylate | Thiethylperazine Malate |
| Thioperamide Maleate | Thioproperazine |
| Thioridazine | Thiothixene |
| Thiothixene, (E)- | Thonzonium Bromide |
| Tioconazole Related Compound A | TMB-8 HCl |
| Tolterodine L-Tartrate | Toremifene Citrate |
| Tramazoline HCl | Trans-U-50488 Methanesulfonate, (±)- |
| Trazodone HCl | Tridihexethyl Chloride |
| Trifluoperazine HCl | Trifluperidol HCl |
| Triflupromazine HCl | Trihexyphenidyl HCl |
| Trimebutine | Trimeprazine Hemi-L-Tartrate |
| Trimipramine Maleate | Tripelennamine HCl |
| Triprolidine HCl | Triprolidine HCl Z Isomer |
| Tropanyl 3,5-Dimethylbenzoate | Tropine 2-(4-Chlorophenoxy)Butanoate, Maleate |
| U-50488 HCl, (-)- | U-62066 |
| UH 232 Maleate, (+)- | Vecuronium Bromide |
| Verapamil HCl | Verapamil Related Compound B |
| Vesamicol HCl | Vinpocetine |
| W-7 HCl | WB-4101 HCl |
| Xylazine | Xylometazoline HCl |

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵ N-enriched nitrogen are within the scope of this invention.

A preferred embodiment of the invention includes the use of at least one compound binding to the sigma receptor for the production of a medicament for the treatment of elevated triglyceride levels. Also preferred is the use of at least one compound binding to the sigma receptor for the production of a medicament for treatment of chylomicronemia, hyperlipoproteinemia, hyperlipidemia (especially mixed hyperlipidemia), hypercholesterolemia, lipoprotein disorders and dysbetalipoproteinemia. An especially preferred embodiment is drawn to the use of at least one compound binding to the sigma receptor for the production of a medicament for the treatment hypertriglyceridemia including both the sporadic and familial disorder (inherited hypertriglyceridemia).
and dysbetalipoproteinemia

Generally the use of compounds binding to the sigma-1 receptor antagonist to produce a medicament for the treatment of metabolic syndrome does not have to cover the treatment of all its aspects. Thus a treatment reducing plasma levels of triglycerides for treating excess triglycerides in plasma (hypertriglyceridemia), does not necessarily include treatment of plasma cholesterol and glucose levels, that may be also concomitantly be elevated (hypercholesterolemia and hyperglycemia, respectively) in metabolic syndrome.

In addition the use of sigma-1 receptor antagonists to produce a medicament for the treatment of hypertriglyceridemia includes also treatment of elevated levels of triglycerides, which exist as a consequence of an abnormal diet or diseases such as diabetes, obesity or any disease or disturbance causing elevations in triglyceride levels.

Finally the use of sigma-1 receptor antagonists to produce a medicament for the treatment of hypertriglyceridemia includes also the treatment of different pathological conditions involving elevated triglyceride levels, such as chylomicronemia, hyperlipoproteinemia, mixed hyperlipidemia and dysbetalipoproteinemia.

The term "salt" is to be understood as meaning any form of the active compound according to the invention in which this assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" is understood in particular, in the context of this invention, as salt (as defined above) formed either with a physiologically tolerated acid, that is to say salts of the particular active compound with inorganic or organic acids which are physiologically tolerated - especially if used on humans and/or mammals - or with at least one, preferably inorganic, cation which are physiologically tolerated - especially if used on humans and/or mammals. Examples of physiologically tolerated salts of particular acids are salts of: hydrochloric acid, hydrobromic acid, sulfuric acid, hydrobromide, monohydrobromide, monohydrochloride or hydrochloride, methiodide, methanesulfonic acid, formic acid, acetic acid, oxalic acid, succinic acid, malic acid, tartaric acid, mandelic acid, fumaric acid, lactic acid, citric acid, glutamic acid, hippuric acid picric acid and/or aspartic acid. Examples of physiologically tolerated salts of particular bases are salts of alkali metals and alkaline earth metals and with NH₄.

The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

In a very preferred embodiment of the invention the compound binding to the sigma receptor used is acting on the sigma receptor as an antagonist.

In a very preferred embodiment of the invention the compound binding to the sigma receptor used is acting on the sigma receptor as an inverse agonist.

In a very preferred embodiment of the invention the compound binding to the sigma receptor used is acting on the sigma receptor as a partial antagonist.

In another possible embodiment of the invention the compound binding to the sigma receptor used is acting on the sigma receptor as an agonist.

In another embodiment of the invention the compound binding to the sigma receptor used is acting on the sigma receptor as a mixed agonist/antagonist, a partial agonist or a partial antagonist.

In another embodiment of the invention the sigma receptor to which the "compound binding to the sigma receptor" is binding to is the sigma-1 receptor. Under this embodiment "Compound/s binding to the sigma receptor" as used in this application is/are defined as having an IC50 value ≤ 5000 nM, more preferably ≤ 1000nM, more preferably ≤500 nM. More preferably, the IC₅₀ value is ≤ 250 nM. More preferably, the IC₅₀ value is ≤ 100 nM. Most preferably, the IC₅₀ value is ≤ 50 nM. Additionally, the wording "Compound/s binding to the sigma receptor", as used in the present application is defined as having at least ≥ 50% displacement using 10 mM radioligand specific for the sigma receptor (e.g. preferably ¹H-pentazocine) whereby the sigma recepor may be any sigma receptor subtype.

In another preferred embodiment of the invention, the compound binding to the sigma receptor, as defined above, has an IC₅₀ value of ≤1000 nM.

In another preferred embodiment of the invention, the compound binding to the sigma receptor, as defined above, has an IC₅₀ value of ≤500 nM.

In another preferred embodiment of the invention, the compound binding to the sigma receptor, as defined above, has an IC₅₀ value of ≤250 nM.

In another preferred embodiment of the invention, the compound binding to the sigma receptor, as defined above, has an IC₅₀ value of ≤100 nM.

In another preferred embodiment of the invention, the compound binding to the sigma receptor, as defined above, has an IC₅₀ value of ≤50 nM.

Most preferably, "compounds highly specific for the sigma receptor" are defined as being "Compound/s binding to the sigma receptor", as defined above, having an IC₅₀ value of ≤100 nM.

In a highly preferred embodiment of the present invention, the compound binding to the sigma receptor as defined above, is binding to the sigma-1 receptor subtype.

In another possible aspect of the invention, the compound binding to the sigma receptor as defined above, may bind to the sigma-2 receptor subtype.

In human therapeutics, the dose administered can be quite low depending on the route of administration and is well known in the art because sigma compounds are known therapeutics.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000 milligrams of active substance to be administered during one or several intakes per day.

Any medicament according to the invention contains the active ingredient as well as optionally at least one auxiliary material and/or additive and/or optionally another active ingredient.

The auxiliary material and/or additive can be specifically selected from conserving agents, emulsifiers and/or carriers for parenteral application. The selection of these auxiliary materials and/or additives and of the amounts to be used depends upon how the pharmaceutical composition is to be applied. Examples include here especially parenteral like intravenous subcutaneous or intramuscular application formulations but which could also be used for other administration routes.

Routes of administration preferably include intramuscular injection, intraveneous injection, subcutaneous injection, sublingual, bucal, patch through skin, oral ingestion, implantable osmotic pump, collagen implants, aerosols or suppository.

Included in this invention are especially also methods of treatments of a patient or a mammal, including men, suffering from metabolic syndrome using compounds binding to the sigma receptor.

In another embodiment the use according to the invention; especially for the production of a medicament for the treatment of lipoprotein disorders; of a compound according to formula II 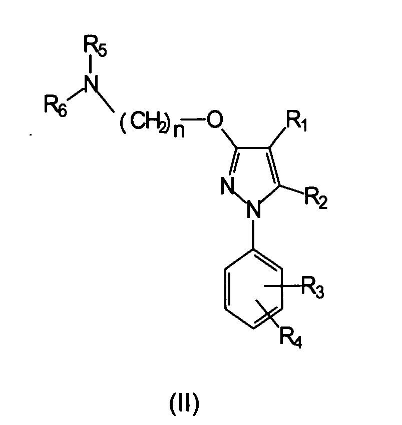 wherein
R₁ is selected from the group formed by hydrogen, substituted or unsubstituted alkyl, substituted or unsubstituted cycloalkyl, substituted or unsubstituted alkenyl, substituted or unsubstituted arylalkyl, substituted or unsubstituted non-aromatic heterocyclyl, substituted or unsubstituted heterocyclylalkyl, -COR₈, -C(O)OR₈, -C(O)NR₈R₉ - C=NR₈, -CN, -OR₈, -OC(O)R₈, -S(O)ₜ-R₈ , -NR₈R₉, -NR₈C(O)R₉, -NO₂, - N=CR₈R₉, or halogen;
R₂ is selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, substituted or unsubstituted heterocyclylalkyl, -COR₈, -C(O)OR₈, - C(O)NR₈R₉ -C=NR₈, -CN, -OR₈, -OC(O)R₈, -S(O)ₜ-R₈, -NR₈R₉,-NR₈C(O)R₉, -NO₂, -N=CR₈R₉, or halogen;
R₃ and R₄ are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, substituted or unsubstituted heterocyclylalkyl, -COR₈, -C(O)OR₈, -C(O)NR₈R₉ -C=NR₈, -CN, -OR₈,-OC(O)R₈, -S(O)ₜ-R₈, -NR₈R₉, -NR₈C(O)R₉, -NO2, -N=CR₈R₉, or halogen, or together they form a fused ring system,
**R₅** and **R₆** are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, substituted or unsubstituted heterocyclylalkyl, -COR₈, -C(O)OR₈, -C(O)NR₈R₉ -C=NR₈, -CN, -OR₈, - OC(O)R₈, -S(O)ₜ-R₈ , -NR₈R₉, -NR₈C(O)R₉, -NO₂, -N=CR₈R₉, or halogen; together form, with the nitrogen atom to which they are attached, a substituted or unsubstituted heterocyclyl group;
n is selected from 1, 2, 3, 4, 5, 6, 7 or 8;
t is 1,2 or 3;
**R₈** and **R₉** are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, or halogen;
or a pharmaceutically acceptable salt, isomer, prodrug or solvate thereof.
or
of a compound of the formula IIB: 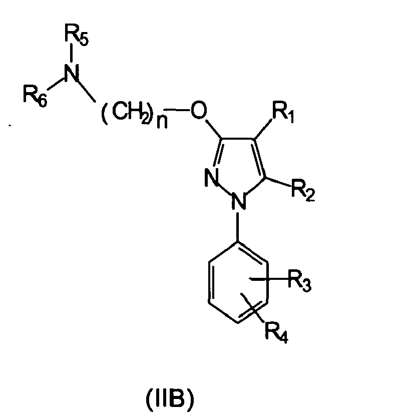 wherein
**R₁** is selected from the group formed by substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted non-aromatic heterocyclyl, substituted or unsubstituted aromatic heterocyclyl, substituted or unsubstituted heterocyclylalkyl, -COR₈, -C(O)OR₈, - C(O)NR₈R₉ -C=NR₈, -CN, -OR₈, -OC(O)R₈, -NR₈R₉, -NR₈C(O)R₉, - NO₂, -N=CR₈R₉ or halogen,
**R₂** is selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, substituted or unsubstituted heterocyclylalkyl, -COR₈, -C(O)OR₈, - C(O)NR₈R₉ -C=NR₈, -CN, -OR₈, -OC(O)R₈, -S(O)ₜ-R₈ , -NR₈R₉, - NR₈C(O)R₉, -NO₂, -N=CR₈R₉, or halogen;
**R₃** and **R₄** are independently selected from the group formed by substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, substituted or unsubstituted heterocyclylalkyl, -COR₈, -C(O)OR₈, -C(O)NR₈R₉ -C=NR₈, -CN, -OR₈, - OC(O)R₈, -S(O)ₜ-R₈ , -NR₈R₉, -NR₈C(O)Rg, -NO2, -N=CR₈R₉, or halogen, or together they form a fused ring system,
**R₅** and **R₆** are independently selected from the group formed by hydrogen, substituted or unsubstituted alkyl, substituted or unsubstituted cycloalkyl, substituted or unsubstituted alkenyl, substituted or unsubstituted aryl, substituted or unsubstituted arylalkyl, substituted or unsubstituted heterocyclyl, substituted or unsubstituted heterocyclylalkyl, -COR₈, -C(O)OR₈, -C(O)NR₈R₉ -C=NR₈, -CN, -OR₈, - OC(O)R₈, -S(O)ₜ-R₈ , -NR₈R₉, -NRgC(O)R₉, -NO₂, -N=CR₈R₉, or halogen; together form, with the nitrogen atom to which they are attached, a substituted or unsubstituted heterocyclyl group;
**n** is selected from 1, 2, 3, 4, 5, 6, 7 or 8;
**t** is 1,2 or 3;
**R₈** and **R₉** are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, or halogen;
or a pharmaceutically acceptable salt, isomer, prodrug or solvate thereof;
is excluded/disclaimed.

In another embodiment the use according to the invention; especially for the production of a medicament for the treatment of lipoprotein disorders, hyperlipidemia, hypertriglyceridemia or hypercholesterolemia,; of compounds according to general formula I 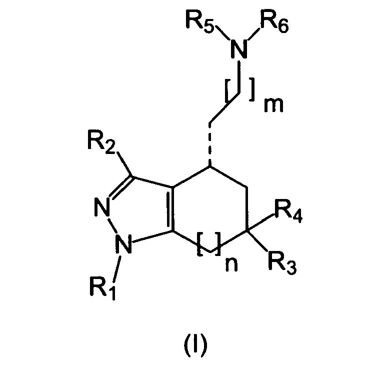 wherein
R₁ is selected from the group formed by hydrogen, susbtituted or unsubstituted alkyl, susbtituted or unsubstituted cycloalkyl, susbtituted or unsubstituted heterocyclyl, susbtituted or unsubstituted aryl, susbtituted or unsubstituted arylalkyl, and susbtituted or unsubstituted heterocyclylalkyl;
R₂ is selected from the group formed by hydrogen, susbtituted or unsubstituted alkyl, susbtituted or unsubstituted cycloalkyl, susbtituted or unsubstituted alkoxy, susbtituted or unsubstituted aryl, susbtituted or unsubstituted heterocyclyl, susbtituted or unsubstituted arylalkyl, and susbtituted or unsubstituted heterocyclylalkyl;
R₃ and R₄ are independently selected from the group formed by hydrogen, susbtituted or unsubstituted alkyl, susbtituted or unsubstituted cycloalkyl, susbtituted or unsubstituted heterocyclyl, susbtituted or unsubstituted aryl, susbtituted or unsubstituted arylalkyl and susbtituted or unsubstituted heterocyclylalkyl or, together, R₃ and R₄ form a 3 to 6 substituted or unsubstituted member ring;
R₅ and R₆ are independently selected from the group formed by hydrogen, susbtituted or unsubstituted alkyl, susbtituted or unsubstituted cycloalkyl, susbtituted or unsubstituted heterocyclyl, susbtituted or unsubstituted aryl, susbtituted or unsubstituted arylalkyl and susbtituted or unsubstituted heterocyclylalkyl or, R₅ and R₆ together, form a substituted or unsubstituted heterocyclyl having 3 to 7 atoms in the ring;
n is selected from 0, 1 and 2;
m is selected from 0, 1, 2, 3, 4;
the dotted line ------ is either a single or a double bond;
with the proviso that when R₁ is phenyl, R₂ is H, the dotted line ------ is a double bond, m is 1, and R₅ and R₆ form a 2,5-dioxopyrrolidine or a 5-ethoxy,2-oxo-pyrrolidine; then R₃ and R₄ are not both at the same time H or methyl;
or a pharmaceutically acceptable salt, isomer, prodrug or solvate thereof
is excluded/disclaimed.

In another embodiment the use according to the invention of compounds according to general formula IA 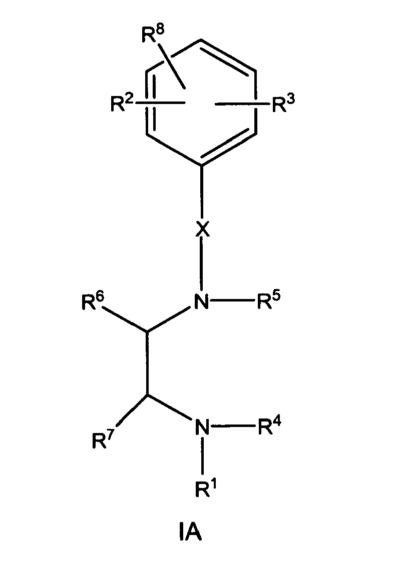 wherein
R¹ is selected from C₁₋₆-Alkyl, saturated or unsaturated, substituted or not substituted, branched or not branched;
R², R³ and R⁸ are independently of each other selected from H; OH, SH, NH₂, C₁₋₆Alkyl, saturated or unsaturated, substituted or not substituted, branched or not branched; Halogen; O-C₁₋₆-Alkyl, saturated or unsaturated, substituted or not substituted, branched or not branched; O-C(O)-C₁₋₆-Alkyl, saturated or unsaturated, substituted or not substituted, branched or not branched; C(O)-O-C₁₋₆-Alkyl, saturated or unsaturated, substituted or not substituted, branched or not branched; or NH-C(O)-C₁₋₆-Alkyl, saturated or unsaturated, substituted or not substituted, branched or not branched;
R⁴ and R⁵ are independently of each other selected from H; OH, SH, NH₂, C₁₋₆Alkyl, saturated or unsaturated, substituted or not substituted, branched or not branched; Halogen; O-C₁₋₆-Alkyl, saturated or unsaturated, substituted or not substituted, branched or not branched;
or
R⁴ and R⁵ taken together are -(CHR⁹)ₙ- forming a ring with n is selected from 1, 2 or 3 and each R⁹ independently selected from H; OH, SH, NH₂, C₁₋₆-Alkyl, saturated or unsaturated, substituted or not substituted, branched or not branched; Halogen; O-C₁₋₆-Alkyl, saturated or unsaturated, substituted or not substituted, branched or not branched;
R⁶ and R⁷ are independently of each other selected from H; OH, SH, NH₂, C₁₋₆-Alkyl, saturated or unsaturated, substituted or not substituted, branched or not branched; Halogen; or O-C₁₋₆-Alkyl, saturated or unsaturated, substituted or not substituted, branched or not branched;
X is -(CHR¹⁰)m-
with m selected from 0, 1, 2, 3 or 4 and
(if applicable) each R¹⁰ independently selected from H; OH, SH, NH₂, C₁₋₆-Alkyl, saturated or unsaturated, substituted or not substituted, branched or not branched; Halogen; O-C₁₋₆-Alkyl, saturated or unsaturated, substituted or not substituted, branched or not branched;
optionally in the form of its racemate, pure stereoisomers, especially enantiomers or diastereomers or in the form of mixtures of stereoisomers, especially enantiomers or diastereomers, in any suitable ratio; in the form shown or in form of the acid or base or in form of a salt, especially a physiologically acceptable salt, or in form of a solvate, especially a hydrate,
is excluded/disclaimed.

The examples and figures in the following section describing pharmacological trials are merely illustrative and the invention cannot be considered in any way as being restricted to these applications.

### Examples

**Rationale:** The present invention provides evidence supporting the use of sigma-1 receptor antagonist to reduce plasma levels of triglycerides. From the experimental point of view, both genetic and pharmacological approaches support the use of sigma-1 receptor antagonist to reduce plasma levels of triglycerides.

### Example 1:

### Genetic approach: Knockout mice deficient for the sigma-1 receptor (σ1^{-/-}) show reduced plasma levels of triglycerides respect to wild type mice.

Male and female mice from the C57BL/6J strain, including wild type and knockout for the sigma-1 receptor, were used in these experiments. The number of animals per group ranged from 16 to 23. Age ranged from 9-15 weeks. All mice had free access to water ad food (standard diet for rodents SAFE A04C; Scientific Animal Food and Engineering, 91360-Villemoisson sur Orge, France; Batch 40123). After a fasting period of 3-5 hours, mice were slightly anesthetized with isofluorane, blood samples were obtained from the retroorbital sinus and plasma was obtained by centrifugation.

Triglyceride levels in plasma were determined using the GPO/peroxidase method. Student t test was applied to determine statistical significance.
The results are presented below. Values are means ± standard deviation.
Units are expressed as mg/100 mL.
- Triglyceride levels in male mice:
   - Wild type (C57BL/6J σ1^{+/+}; n=20): 105.9 ± 39.39
   - Knockout sigma-1 receptor (C57BL/6J σ1^{-/-}; n=16): 71.5 ± 18.58 **
      ^{**} p<0.01 compared to the control wild type group
- Triglyceride levels in female mice
   - Wild type (C57BL/6J σ1^{+/+}; n=20): 76.7 ± 29.08
   - Knockout sigma-1 receptor (C57BL/6J σ1^{-/-}; n=23): 58.2 ± 12.41 *
      * p<0.05 compared to the control wild type group

### Example 2:

### Pharmacologic approach: Treatment of obese (diet-induced obesity) wild type mice with a sigma-1 receptor antagonist results in a significant reduction in the concentration of triglycerides in blood.

Wild type male mice from the C57BL/6J strain were used in these experiments. Mice of 8-10 weeks of age were fed for two months with high fat diet (49 % fat content; Harlan Ibérica, TD97366). Treatment was administered subcutaneously, once a day, for 9 days. Treated animals received daily (for 9 days) a single dose of 50 mg/kg of BD-1063, a sigma-1 receptor antagonist. Control animals received daily vehicle (saline). During the period of treatment, mice had free access to water and food (high fat diet). At the end of the treatment, on day 10, blood samples were obtained through intracardiac puncture and triglyceride levels were measured using the Cholestech LDX blood analyzer. Student t test was applied to determine statistical significance.
The results are presented below. Values are means ± standard deviation.
Units are expressed as mg/100 mL.
- Triglyceride levels:
   - Control untreated (vehicle treated) mice: 91.75 ± 32.88
   - Treated (50 mg/Kg BD-1063, s.c., once a day, 9 days): 56.8 ± 9.41 *
      * p<0.05 compared to the control wild type group

### References

Fredrickson DS. An international classification of hyperlipidemias and hyperlipoproteinemias. Ann Intern Med. 1971 Sep;75(3):471-2.

Beaumont JL, Carlson LA, Cooper GR, Fejfar Z, Fredrickson DS, Strasser T. Classification of hyperlipidaemias and hyperlipoproteinaemias. Bull World Health Organ. 1970;43(6):891-915.

## Claims

1. Use of at least one compound binding to the sigma receptor for the production of a medicament for the treatment of metabolic syndrome.

2. Use, according to claim 1, **characterized in that** said compound may be in neutral form, the form of a base or acid, in the form of a salt, preferably a physiologically acceptable salt, in the form of a solvate or of a polymorph and/or in the form of in the form of its racemate, pure stereoisomers, especially enantiomers or diastereomers or in the form of mixtures of stereoisomers, especially enantiomers or diastereomers, in any suitable mixing ratio.

3. Use, according to claim 1, **characterized in that** said compound binding to the sigma receptor has an IC50 value of ≤5000 nM.

4. Use, according to claim 1, **characterized in that** said compound binding to the sigma receptor has an IC50 value of ≤1000 nM.

5. Use, according to claim 1, **characterized in that** said compound binding to the sigma receptor has an IC50 value of ≤500 nM.

6. Use, according to claim 1, **characterized in that** said compound binding to the sigma receptor has an IC50 value of ≤250 nM.

7. Use, according to claim 1, **characterized in that** said compound binding to the sigma receptor has an IC50 value of ≤100 nM.

8. Use, according to claim 1, **characterized in that** said compound binding to the sigma receptor has an IC50 value of ≤50 nM.

9. Use, according to any of claims 1 to 8, **characterized in that** said compound binding to the sigma receptor used is acting on the sigma receptor as an antagonist.

10. Use, according to any of claims 1 to 8, **characterized in that** said compound binding to the sigma receptor used is acting on the sigma receptor as a partial antagonist.

11. Use, according to any of claims 1 to 8, **characterized in that** said compound binding to the sigma receptor used is acting on the sigma receptor as an inverse agonist.

12. Use, according to any of claims 1 to 11, **characterized in that** said compound binding to the sigma receptor is binding to the sigma-1 receptor subtype.

13. Use, according to claim 1, **characterized in that** said compound binding to the sigma receptor is selected from the group consisting of:
| (-)-Cyanopindolol hemifumarate | (-)-SPARTEINE SULFATE PENTAHYDRATE |
|---|---|
| (+)-HIMBACINE | (2-Dibutylamino-Ethyl)-Carbamic Acid 2-(4-Benzofuran-2-Ylmethyl-Piperazin-1-YI)-Ethyl Ester |
| (4-[1,2,3]Thiadiazol-4-YI-Benzyl)-Carbamic Acid 1-(3-Methoxy-2-Nitro-Benzyl)-Piperidin-3-Ylmethyl Ester | (S)-Methamphetamine HCl |
| [1-(9-Ethyl-9H-Carbazol-3-Ylmethyl)-Pyrrolidin-3-YI]-Carbamic Acid 1-(3-Benzyloxy-4-Methoxy-Benzyl)-Piperidin-3-Ylmethyl Ester | [1-(9-Ethyl-9H-Carbazol-3-Ylmethyl)-Pyrrolidin-3-YI]-Carbamic Acid 2-(Tert-Butoxycarbonyl-Naphthalen-1-Ylmethyl-Amino)-Ethyl Ester |
| [4-(4-Ethyl-3,5-Dimethyl-Pyrazol-1-YI)- Phenyl]-[4-(3-Phenyl-Allyl)-Piperazin-1-YI]-Methanone | 1-(1,2-Diphenylethyl)Piperidine Maleate, (+/-) |
| 1-(1-Naphthyl)Piperazine HCl | 1-(3-Chlorophenyl)Piperazine HCl |
| 1-(4-Bromo-Benzenesulfonyl)-4-(2-Tert-Butylsulfanyl-Benzyl)-Piperazine | 2-(2-{[1-(3-Chloro-Benzyl)-Pyrrolidin-3-Yl]-Methyl-Carbamoyl}-2-Methyl-Propyl)-4,6-Dimethyl-Benzoic Acid |
| 2-Chloro-11-(4-Methylpiperazino)Dibenz[B, F]Oxepin Maleate | 3,3'-Diethylthiacarbocyanine Iodide |
| 3-Mercapto-2-Methylpropanoic Acid 1,2- Diphenylethylamine Salt | 3-Quinuclidinyl Benzilate |
| 3-Tropanyl-3,5-Dichlorobenzoate | 3-Tropanyl-Indole-3-Carboxylate HCl |
| 4-(1H-Indol-4-Yl)-Piperazine-1-Carboxylic Acid 2-(5-Bromo-2-Ethoxy- | 4-(2-Tert-Butylsulfanyl-Benzyl)-Piperazine-1-Carboxylic Acid 2- |
| Phenylamino)-Cyclohexylmethyl Ester | Thiophen-2-Yl-Ethyl Ester |
| 4-(3,5-Dimethoxy-Phenyl)-Piperazine-1-Carboxylic Acid 1-(2-Fluoro-Benzyl)-Piperidin-2-Ylmethyl Ester | 4-(3-Nitro-5-Sulfamoyl-Thiophen-2-Yl)-Piperazine-1-Carboxylic Acid 1-(2-Fluoro-5-Methoxy-Benzyl)-Piperidin-3-Ylmethyl Ester |
| 4-(4-Fluorobenzoyl)-1-(4-Phenylbutyl)Piperidine Oxalate | 4-(5-Trifluoromethyl-Pyridin-2-Yl)-Piperazine-1-Carboxylic Acid Pent-2-Ynyl Ester |
| 4,4'-Bis[4-(P-Chlorophenyl)-4-Hydroxypiperidino]Butyrophenone | 4-[1-(4-Chlorobenzyl)-4-(benzylpiperidin-4-yl]-2-hydroxy-4-oxobut-2-enoic acid |
| 4-Bromo-N-[1-(9-Ethyl-9H-Carbazol-3- Ylmethyl)-Pyrrolidin-3-Yl]-2-Trifluoromethoxy-Benzenesulfonamide | 4'-Chloro-3-Alpha-(Diphenylmethoxy)Tropane HCl |
| 4-Furan-2-Ylmethyl-Piperazine-1-Carboxylic Acid 2-{4-[3-(2-Trifluoromethyl-Phenothiazin-10-Yl)-Propyl]-Piperazin-1-Yl}-Ethyl Ester | 4-Methoxy-N-[1-(7-Methoxy-Benzo[1,3]Dioxol-5-Ylmethyl)-Pyrrolidin-3-Yl]-Benzenesulfonamide |
| 5-(N-Ethyl-N-Isopropyl)-Amiloride | 7-Hydroxy-DPAT HBr, (±)- |
| 8-Hydroxy-DPAT HBr, (R)-(+)- | 8-Hydroxy-DPAT HBr, S(-)- |
| 9-[4-({[4'-(trifluoromethyl)-1,1'-biphenyl-2-yl]carbonyl}amino)piperidin-1-yl]-N-(2,2,2-trifluoroethyl)-9H-fluorene-9-carboxamide | Acepromazine Maleate |
| Acetophenazine Maleate | Acrinol |
| Ajmaline | Alaproclate HCl |
| Aloe-Emodin | Alprenolol D-Tartrate Salt Hydrate |
| Alprenolol HCl | AMI-193 |
| Aminobenztropine | Amiodarone HCl |
| Amodiaquine HCl | Amorolfine HCl |
| Amoxapine | Anileridine HCl |
| Anisotropine Methylbromide | Anpirtoline |
| ARC 239 DiHCl | Astemizole |
| Auramine O HCl | Azaperone |
| Azatadine Maleate | Azelastine HCl |
| Bamethan sulfate | BD 1008 DiHBr |
| BD-1047 | BD-1063 |
| Benextramine TetraHCl | Benfluorex HCl |
| Benidipine HCl | Benoxathian HCl |
| Benoxinate HCl | Benperidol |
| Benproperine Phosphate | Benzododecinium bromide |
| Benzphetamine HCl | Benztropine Mesylate |
| Benzydamine HCl | Bephenium Hydroxynaphthoate |
| Bepridil HCl | Berberine chloride |
| Betaxolol HCl | Bifemelane |
| BMY 7378 DiHCl | Bopindolol Malonate |
| BP 554 Maleate | Bromhexine HCl |
| Bromodiphenhydramine HCl | Bromperidol |
| Brompheniramine Maleate | BTCP HCl |
| Buclizine HCl | Buflomedil HCl |
| Bupropion HCl | Buspirone HCl |
| Butacaine Sulfate | Butaclamol HCl, (±)- |
| Butenafine HCl | Butoconazole Nitrate |
| BW 723C86 HCl | Carbetapentane Citrate |
| Carbinoxamine Maleate | Carpipramine DiHCl DiH2O |
| Carvedilol | Cephapirin Benzathine |
| CGS-12066A Maleate | Chloroprocaine HCl |
| Chloroquine Phosphate | Chlorpheniramine Maleate |
| Chlorphenoxamine HCl | Chlorpromazine HCl |
| Chlorprothixene | Cinanserin HCl |
| Cinnarizine | Cirazoline HCl |
| Cis-(+/-)-N-Methyl-N-[2-(3,4-Dichlorophenyl)Ethyl]-2-(1-Pyrrolidinyl)Cyclohexamine DiHBr | Cis(Z)-Flupentixol DiHCl |
| Cisapride Hydrate | Citalopram HBr |
| Clebopride Maleate Salt | Clemastine Fumarate |
| Clemizole HCl | Clenbuterol HCl |
| Clidinium Bromide | Clobenpropit 2HBr |
| Clofazimine | Clofilium Tosylate |
| Clomiphene Citrate | Clomiphene Related Compound A |
| Clomipramine | Cloperastine HCl |
| Clorgyline HCl | Clozapine |
| CONESSINE | Cyclizine |
| Cyclobenzaprine HCl | Cycloheximide |
| Cyproheptadine HCl | Darrow Red HCl |
| Demecarium Bromide | Denatonium Benzoate |
| Deptropine Citrate | Desloratadine |
| Dexbrompheniramine Maleate | Dexchlorpheniramine Maleate |
| Dexfenfluramine HCl | Dibucaine HCl |
| Dicyclomine HCl | Diethylpropion HCl |
| Dimethisoquin HCl | Dimetindene Maleate |
| Diphemanil Methylsulfate | Diphenidol HCl |
| Diphenoxylate HCl | Diphenylpyraline HCl |
| Dipropyldopamine HBr | Dobutamine HCl |
| Donepezil HCl | Doxepin HCl |
| Droperidol | Duloxetine |
| Dyclonine HCl | Ebastine |
| Econazole Nitrate | Epinastine HCl |
| Ethaverine HCl | Ethopropazine HCl |
| Eticlopride HCl, S(-)- | Etofenamate |
| Etonitazenyl Isothiocyanate | Femoxetine HCl |
| Fenfluramine HCl | Fentanyl Citrate |
| Fenticonazole Nitrate | Fipexide HCl |
| Flavoxate HCl | Flunarizine diHCl |
| Fluoxetine Related Compound B | Fluperlapine |
| Fluphenazine Decanoate DiHCl | Fluphenazine Enanthate DiHCl |
| Fluphenazine HCl | Fluphenazine N-Mustard DiHCl |
| Flurazepam Related Compound C | Fluspirilene |
| Fluvoxamine Maleate | GBR 12783 DiHCl |
| GBR 12909 DiHCl | GBR 13069 DiHCl |
| GBR-12935 DiHCl | GR 89696 Fumarate |
| Guanabenz Acetate | Guanadrel Sulfate |
| Guanethidine Sulfate | Halofantrine HCl |
| Haloperidol | HEAT HCl |
| Hexylcaine HCl | Hycanthone |
| Hydroxychloroquine Sulfate | Hydroxyzine HCl |
| Hyoscyamine Sulfate | IBZM, S(-)- |
| ICI-199,441 HCl | Ifenprodil Tartrate |
| Imipramine HCl | Indatraline HCl |
| lofetamine HCl | Irinotecan HCl |
| Isamoltane Hemifumarate | Isopromethazine HCl |
| Isoxsuprine HCl | Ketanserin L-Tartrate |
| Ketoconazole | Ketotifen Fumarate Salt |
| L-693,403 Maleate | L-741,626 |
| L-741,742 HCl | L-745,870 TriHCl |
| Labetalol HCl | Levetimide HCl, R(-) |
| Levobunolol HCl | Lidoflazine |
| Lisuride Hydrogen Maleate, R(+)- | Lobeline HCl |
| lomerizine diHCl | Loperamide HCl |
| Loxapine Succinate | LY-53,857 Maleate |
| Maprotiline HCl | Mazindol |
| MDL 12,330A HCl | Mebhydroline 1,5-naphthalendisulfonate Salt |
| Meclizine HCl | Mefloquine HCl |
| Meprylcaine HCl | Mesoridazine Besylate |
| Metaphit Methanesulfonate | Metergoline |
| Methantheline Bromide | Methdilazine |
| Methiothepin Mesylate | Methixene HCl |
| Methoctramine | Methotrimeprazine Maleate |
| Methylene Violet 3Rax HCl | Metipranolol |
| Mexiletine HCl | Mianserin HCl |
| Miconazole | ML-9 HCl |
| Morantel Hydrogen L-Tartrate | MR 16728 HCl |
| N-(2-Chloroethyl)-N-Ethyl-2-Bromobenzylamine HCl | N'-[2-(Benzo[1,2,5]Thiadiazole-4-Sulfonylamino)-Acetyl]-Hydrazinecarboxylic Acid 2-(2-{4-[(4-Chloro-Phenyl)-Phenyl-Methyl]-Piperazin-1 -Y)}-Ethoxy)-Ethy) Ester |
| Nafronyl Oxalate Salt | Naftifine |
| Naftopidil diHCl | Naltriben Mesylate |
| NAN-190 HBr | NE-100 |
| Nefazodone | Nefopam HCl |
| Nicardipine HCl | Nicergoline |
| Niguldipine HCl, (+/-)- | Nisoxetine HCl |
| Nortriptyline HCl | Nylidrin HCl |
| Octoclothepin Maleate, (±)- | Orphenadrine Citrate |
| Oxamniquine | Oxamniquine Related Compound A |
| Oxamniquine Related Compound B | Oxatomide |
| Oxiconazole Nitrate | Oxybutynin HCl |
| Panaxatriol | PAPP |
| Paroxetine | Paxilline |
| p-Chlorobenzhydrylpiperazine | Penbutolol Sulfate |
| Pentamidine Isethionate | Pentazocine, (±)- |
| Pergolide Methanesulfonate | Perhexiline Maleate Salt |
| Perospirone | Perphenazine |
| Perphenazine Sulfoxide | Phenamil Methanesulfonate |
| Phencyclidine HCl | Phenosafranin HCl |
| Phenoxybenzamine HCl | Phenyltoloxamine Citrate Salt |
| Piboserod | Pimozide |
| Pinacyanol Chloride | Pindobind, (+/-)- |
| Piperacetazine | Piperazine-1,4-Dicarboxylic Acid Benzyl Ester 2-[4-(4-Dimethylamino-Benzyl)-Piperazin-1-Yl]-Ethyl Ester |
| Piperidolate HCl | Pirenperone |
| PPHT HCl, (±)- | Pramoxine HCl |
| Prenylamine Lactate Salt | Pridinol Methanesulfonate Salt |
| Prochlorperazine Maleate | Procyclidine HCl |
| Proflavine Hemisulfate Salt | Progesterone |
| Promazine HCl | Promethazine HCl |
| Propafenone HCl | Proparacaine HCl |
| Propericyazine | Propiomazine |
| Propranolol HCl | Protokylol |
| Protriptyline HCl | Pyrilamine Maleate |
| Pyrimethamine | Pyrrolidine-1,2-Dicarboxylic Acid 1-[1-(4-Allyloxy-Benzyl)-Piperidin-2-Ylmethyl] Ester 2-Benzyl Ester |
| Pyrvinium Pamoate | Quetiapine Fumarate |
| Quinacrine HCl | Quinaldine Red |
| Quipazine Dimaleate | Quipazine, 6-Nitro-, Maleate |
| Raloxifene | Rimantadine HCl |
| Risperidone | Ritanserin |
| Ritodrine HCl | RS 23597-190 HCl |
| RS 67333 HCl | RS 67506 HCl |
| Safranin O HCl | Salmeterol |
| SB203186 | SCH-23390 HCl, R(+)- |
| Sertaconazole Nitrate | Sertindole |
| Sertraline | Sibutramine HCl |
| SKF-525A HCl | SKF-96365 HCl |
| SNC 121 | Spiperone HCl |
| Sufentanil | T-226296 |
| Tamoxifen Citrate | Tamsulosin HCl |
| Tegaserod Maleate | Terbinafine HCl |
| Terconazole | Terfenadine |
| Terfenadine Related Compound A | Tetracaine HCl |
| Tetrindole Mesylate | Thiethylperazine Malate |
| Thioperamide Maleate | Thioproperazine |
| Thioridazine | Thiothixene |
| Thiothixene, (E)- | Thonzonium Bromide |
| Tioconazole Related Compound A | TMB-8 HCl |
| Tolterodine L-Tartrate | Toremifene Citrate |
| Tramazoline HCl | Trans-U-50488 Methanesulfonate, (±)- |
| Trazodone HCl | Tridihexethyl Chloride |
| Trifluoperazine HCl | Trifluperidol HCl |
| Triflupromazine HCl | Trihexyphenidyl HCl |
| Trimebutine | Trimeprazine Hemi-L-Tartrate |
| Trimipramine Maleate | Tripelennamine HCl |
| Triprolidine HCl | Triprolidine HCl Z Isomer |
| Tropanyl 3,5-Dimethylbenzoate | Tropine 2-(4-Chlorophenoxy)Butanoate, Maleate |
| U-50488 HCl, (-)- | U-62066 |
| UH 232 Maleate, (+)- | Vecuronium Bromide |
| Verapamil HCl | Verapamil Related Compound B |
| Vesamicol HCl | Vinpocetine |
| W-7 HCl | WB-4101 HCl |
| Xylazine | Xylometazoline HCl |

14. Use, according to claim 1, **characterized in that** the medicament produced is for the treatment of elevated triglyceride levels, chylomicronemia, hyperlipoproteinemia; hyperlipidemia, especially mixed hyperlipidemia; hypercholesterolemia, lipoprotein disorders and dysbetalipoproteinemia.

15. Use, according to claim 1, **characterized in that** the medicament produced is for the treatment hypertriglyceridemia including both the sporadic and familial disorder, inherited hypertriglyceridemia.
